# EUROPÄISCHE PATENTSCHRIFT

(11) **EP 2 488 166 B1**
(45) Veröffentlichungstag und Bekanntmachung des Hinweises auf die Patenterteilung: **12.04.2017**
(21) Anmeldenummer: 10734917.7
(22) Anmeldetag: 14.07.2010
(51) Int. Cl.: A61K 9/70

(54) **TRANSDERMALE THERAPEUTISCHE SYSTEME ENTHALTEND 4-N-BUTYLRESORCIN**
TRANSDERMAL THERAPEUTIC SYSTEMS CONTAINING 4-N-BUTYLRESORCINOL
SYSTÈMES THÉRAPEUTIQUES TRANSDERMIQUES CONTENANT DE LA 4-N-BUTYLRÉSORCINE

(30) Priorität: 09.10.2009 DE 102009048973
(43) Veröffentlichungstag der Anmeldung: 22.08.2012
(73) Patentinhaber: Beiersdorf AG, 20253 Hamburg (DE)
(72) Erfinder: SCHERNER, Cathrin, 22844 Norderstedt (DE); WÖLLER, Karl-Heinz, 20257 Hamburg (DE); WOLBER, Rainer, 22397 Hamburg (DE); KOLBE, Ludger, 21255 Dohren (DE)
(86) Internationale Anmeldenummer: PCT/EP2010/004272
(87) Internationale Veröffentlichungsnummer: WO 2011/042077

(56) Entgegenhaltungen:
- EP-A1- 0 341 664
- WO-A1-2009/156676
- WO-A1-2010/049462

## Beschreibung

Die vorliegende Erfindung betrifft transdermale therapeutische Systeme, selbstklebende flächige Bandagen, insbesondere kosmetische und medizinische Pflaster, enthaltend 4-n-Butylresorcin.

Für die Pigmentierung der Haut verantwortlich sind die Melanozyten, welche in der untersten Schicht der Epidermis, dem Stratum basale, neben den Basalzellen als - je nach Hauttyp entweder vereinzelt oder aber mehr oder weniger gehäuft auftretende - pigmentbildende Zellen vorzufinden sind.

Melanozyten enthalten als charakteristische Zellorganellen Melanosomen, in denen das Melanin gebildet wird. Unter anderem bei Anregung durch UV-Strahlung wird verstärkt Melanin gebildet. Dieses wird über die lebenden Schichten der Epidermis (Keratinozyten) letztlich in die Hornschicht (Corneozyten) transportiert und ruft eine mehr oder weniger ausgeprägte bräunliche bis braun-schwarze Hautfarbe hervor.

Melanin wird als Endstufe eines oxidativen Prozesses gebildet, in welchem Tyrosin unter Mitwirkung der Enzyms Tyrosinase über mehrere Zwischenstufen zu den braun bis braunschwarzen Eumelaninen (DHICA- und DHI-Melanin) bzw. unter Beteiligung von schwefelhaltigen Verbindungen zum rötlichen Phäomelanin umgewandelt wird. DHICA- und DHI-Melanin entstehen über die gemeinsamen Zwischenstufen Dopachinon und Dopachrom. Letzteres wird, teilweise unter Beteiligung weiterer Enzyme, entweder in Indol-5,6-Chinon-Carbonsäure oder in Indol-5,6-Chinon umgesetzt, woraus die beiden genannten Eumelanine entstehen.

Die Entstehung von Phäomelanin läuft unter anderem über die Zwischenprodukte Dopachinon und Cysteinyldopa. Gesteuert wird die Expression der Melanin-synthetisierenden Enzyme durch einen spezifischen Transkriptionsfaktor (microphthalmia- associated transcription factor, MITF). Neben den beschriebenen enzymatischen Prozessen der Melanin-Synthese sind in den Melanosomen noch weitere Proteine für die Melanogenese von Bedeutung. Eine wichtige Rolle scheint hier dem sogenannten p-Protein zuzukommen, wobei die exakte Funktion noch unklar ist.

Neben dem zuvor beschriebenen Prozeß der Melanin-Synthese in den Melanozyten, ist bei der Pigmentierung der Haut auch der Transfer der Melanosomen, deren Verbleib in der Epidermis sowie deren Abbau und der Abbau des Melanins von entscheidender Bedeutung. Es konnte gezeigt werden daß für den Transport der Melanosomen aus den Melanozyten in die Keratinozyten der PAR-2-Rezeptor bedeutsam ist (M. Seiberg et al., 2000, J. Cell. Sci., 113:3093-101).

Ferner haben Größe und Form der Melanosomen Einfluß auf ihre lichtstreuenden Eigenschaften und somit das farbliche Erscheinungsbild der Haut. So findet man bei Schwarzafrikanern verstärkt große spheroidale, einzeln vorliegende Melanosomen, während man bei Kaukasiern eher kleinere, in Gruppen vorkommende Melanosomen vorfindet.

Probleme mit Hyperpigmentierung der Haut haben vielfältige Ursachen bzw. sind Begleiterscheinungen vieler biologischer Vorgänge, z.B. UV-Strahlung (z.B. Sommersprossen, *Ephelides*), genetische Disposition, Fehlpigmentierung der Haut bei der Wundheilung bzw. - vernarbung (postinflammatorische Hyperpigmentierung) oder der Hautalterung (z.B. *Lentigines seniles*).

Nach entzündlichen Reaktionen reagiert das Pigmentierungssystem der Haut mit teilweise entgegengesetzten Reaktionen. Es kann sowohl zu postinflammatorischen Hyper- wie auch Hypopigmentierungen kommen. Postinflammatorische Hypomelanosen treten u. a. häufig in Verbindung mit Atopie, Lupus erythematosus und Psoriasis auf. Die unterschiedlichen Reaktionsformen des Pigmentierungssystems der menschlichen Haut in Folge entzündlicher Erscheinungen sind nur sehr unvollständig verstanden.

Probleme mit postinflammatorischer Hyperpigmentierung treten häufig bei dunkleren Hauttypen auf. Insbesondere bei männlichen Farbigen ist das Problem der *Pseudofollikulitis barbae* bekannt, das mit kosmetisch unerwünschten Fehlpigmentierung einhergeht bzw. diese nach sich zieht. Auch Formen von Melasma, welche insbesondere bei Frauen asiatischer Zugehörigkeit im Gesicht und im Dekolleté - Bereich auftreten, sowie verschiedene Formen der unregelmäßigen Pigmentierung der Haut werden zu den postinflammatorischen Hyperpigmentierungen gezählt. Ferner werden auch dunkle Augenringe als eine Form postinflammatorischen Hyperpigmentierungen angesehen, wobei die zugrunde liegende Entzündung meist subklinisch abläuft.

In vielen Fällen werden derartige postinflammatorische Fehlpigmentierung durch Einwirkung von Sonnenlicht (UV-Licht) noch verstärkt, ohne daß es zu einer UV-induzierten Entzündung (Sonnenbrand) kommt.

Es sind Wirkstoffe und Zubereitungen bekannt, welche der Hautpigmentierung entgegenwirken. Im praktischen Gebrauch sind im wesentlichen Präparate auf der Grundlage von Hydrochinon, welche aber einesteils erst nach mehrwöchiger Anwendung ihre Wirkung zeigen, deren übertrieben lange Anwendung andererseits aus toxikologischen Gründen bedenklich ist. Von Albert Kligman et al. wurde eine sogenannte Triformula entwickelt, die eine Kombination aus 0,1% Tretinoin, 5,0% Hydrochinon, 0,1% Dexamethason darstellt (A. Kligman, 1975, Arch. Dermatol., 111:40-48). Allerdings ist auch diese Formulierung wegen möglicher irreversibler Veränderungen im Pigmentierungssystem der Haut sehr umstritten.

Ferner finden hautschälende Methoden (chemische und mechanische "Peelings") Anwendung, die jedoch häufig entzündliche Reaktionen nach sich ziehen und aufgrund danach eintretender postinflammatorischer Hyperpigmentierungen sogar zu stärkerer statt verminderter Pigmentierung führen können. All diese gängigen Verfahren, die auch zur Behandlung von postinflammatorischen Hypergigmentierungen angewendet werden, zeichnen sich durch entscheidende Nebenwirkungen aus.

Kosmetische Zubereitungen mit 4-n-Butylresorcin sind bekannt, beispielsweise aus der EP 1 490017.

4-n-Butylresorcin, CAS[18979-61-8], ist durch die chemische Struktur 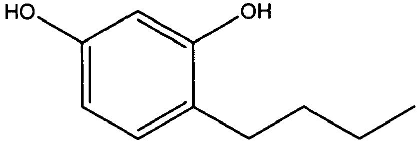 gekennzeichnet.

4-n-Butylresorcin wird auch Rucin oder Lucin genannt. Es hemmt die Melaninproduktion, indem es das für die Melaninsynthese (Melanogenese) erforderliche Enzym, die Tyrosinase, hemmt. Es wird zunächst die Melaninproduktion gehemmt, dann die Produktion des schwarzen Melanins, das für die intensive Färbung der Pigmentflecken verantwortlich ist, blockiert.

4-n-Butylresorcin hat den formulatorischen Nachteil, daß es dazu neigt, sich - und kosmetische oder dermatologische Zubereitungen, es enthaltend - zu verfärben.

Transdermale therapeutische Systeme ("TTS") sind an sich bekannt. Nachteilig bei TTS, ist, daß üblicherweise während der Applikationszeit nur ca. 10 % bis 20 % der Wirkstoffbeladung des Pflasters freigesetzt werden. (Kommentar zum Europäischen Arzneibuch, Wissenschaftliche Verlagsgesellschaft Stuttgart, Stand: Aktualisierungslieferung 2009).

Ziel der nachfolgenden Erfindung war es also, den Nachteilen des Standes der Technik Abhilfe zu verschaffen.

Es war überraschend und für den Fachmann nicht vorhersehbar, daß Übelstände des Standes der Technik beseitigt werden durch transdermale therapeutische Systeme, welche als Wirstoff 4-n-Butylresorcin enthalten.

Zur Erarbeitung eines wirksamen selbstklebenden Pflasters 4-Butyl-resorcin enthaltend mit optimierter Freisetzung zur Behandlung von Pigmentstörungen der Haut wurden unterschiedliche selbstklebende Matrixsysteme mit 1 % des Wirkstoffes hergestellt und auf ihre Freisetzungseigenschaften nach 24 Stunden auf Schweinehaut mittels Franzscher Zellen untersucht:
Eine unpolare Matrix auf Basis von Synthese- und Naturkautschuk (KA)
Ein polarer feucht klebender Film auf Basis Polyacrylsäure / Polyvinylalkohol (FKF)
Eine unpolare Matrix auf Basis eines Polyacrylsäurecopolymers (PAC)
Eine polare wasserfreie Gelmatrix auf Basis Polyacrylsäure / Polyvinylpyrrolidon (WFG)
Eine unpolare Polyisobutylenmatrix (PIB)
Ein polare Wassergelmatrix auf Basis Agar Agar / Polyacrylsäure (WG)

Ergebnisse der Wirkstofffreisetzung unterschiedlicher Matrixsysteme:

| (KA) | (FKF) | (PAC) | (WFG) | (PIB) | (WG) |
|---|---|---|---|---|---|
| 7,4 % | 17,8 % | 18,1 % | 20,6 % | 22,2 % | 34,0 % |

Basierend auf obigen ersten Ergebnissen wurden die unpolare Polyisobutylenmatrix und die polare Wassergelmatrix für weitere Optimierungsuntersuchungen hinsichtlich der Freisetzung von 4-n-Butylresorcin ausgewählt.

Die Wirkstofffreisetzung kann vorteilhaft durch die Schichtdicke des TTS gesteuert werden.

Es wurden von beiden Systemen, PIB und WG, jeweils Muster mit 1,00; 0,75; 0,50; 0,30 und 0,15 mm Schichtdicke hergestellt und untersucht.

Ergebnisse der Wirkstofffreisetzung unterschiedlicher Matrixschichtdicken:

| | 1,00 mm | 0,75 mm | 0,50 mm | 0,30 mm | 0,15 mm |
|---|---|---|---|---|---|
| (PIB) | 22,2 % | 27,4 % | 26,7 % | 42,7 % | 46,4 % |
| (WG) | 34,0 % | 48,3 % | 41,8 % | 66,2 % | 56,8 % |

Als Kompromiss zwischen prozentualer Wirkstofffreisetzung, absoluter freigesetzter Wirkstoffmenge und Handhabbarkeit des fertigen selbstklebenden Pflasters wird bei beiden Matrixsystemen eine Schichtdicke zwischen 1,00mm und 0,01mm, bevorzugt 0,50 und 0,20mm und ganz besonders bevorzugt von 0,30 mm erachtet. Insbesondere das selbstklebende Patch basierend auf einem erfindungsgemäßen polaren Wassergel zeigt mit rund 65 % des Wirkstoffs eine im Vergleich zu üblichen Pflasteranwendungen überraschend hohe Freisetzung.

Um das PIB-Matrixsystem ebenfalls in diesen Bereich der Wirkstofffreisetzung zu bringen wurden weitere Versuche mittels Einarbeitung von üblichen Additiven zur Erhöhung der Hydrophile der Matrix sowie auch von üblichen Penetrationsbeschleunigern durchgeführt.

Die Einarbeitung von 35 % Cellulose in die PIB Matrix ergab keinen signifikanten Unterschied in der Wirkstofffreisetzung gegenüber einer nicht mit Cellulose gefüllten Matrix.

Als übliche Penetrationsbeschleuniger wurden dann jeweils 5 % Isopropylpalmitat (IPP) bzw. 5 % Isopropylmyristat (IPM) in eine entsprechende PIB-Matrix eingearbeitet und die Freisetzung an 4-n-Butylresorcin aus den daraus hergestellten Endprodukten von 0,30 mm Schichtdicke bestimmt.

Ergebnisse der Wirkstofffreisetzung PIB Matrix unterschiedlicher Penetrationsbeschleuniger:

| | PIB-Matrix plus 5 % IPP | PIB-Matrix plus 5 % IPM |
|---|---|---|
| Freisetzung von 4-n-Butylresorcin | 33,2 % | 61,3 % |

Überraschenderweise zeigte der Zusatz von 5 % IPM eine fast doppelt so hohe Wirkstofffreisetzung aus der PIB-Matrix wie der Zusatz des homologen Penetrationsbeschleunigers IPP.

Mit rund 61 % Freisetzung 4-n-Butylresorcin aus der unpolaren PIB-Matrix konnte eine analoge Größenordnung erreicht werden wie aus der polaren Wassergel-Matrix mit rund 65 %.

Beispiele für die Herstellung polarer selbstklebender Wassergel-Matrices sind in DE 102 60 872 beschrieben.

Beispiele für die Herstellung unpolarer selbstklebender PIB-Matrices sind in EP 1335755 beschrieben.

Als Trägermaterialien für erfindungsgemäße polare wie unpolare selbstklebende Patches mit optimierter Wirkstofffreisetzung zur Behandlung von Pigmentstörungen der Haut eignen sich alle gängigen flächigen Schichtmaterialien wie beispielsweise Gewebe, Folien, Vliese etc. Bei letztgenannten Trägermaterialien sind insbesondere sogenannte Non-wovens von geringer Schichtdicke vorteilhaft, da diese auf den Matrices gegenüber der Haut optisch kaum auffällig sind. Besonders vorteilhaft ist die Verwendung von sehr dünnen, Schichtdicke unter 100 µm, flexiblen und durchsichtigen bis translucenten Polymerfolien, insbesondere solchen aus wässriger Dispersion hergestellten Polyurethanfolien.

Es können aber auch Folien aus allen anderen bekannten Polymerfilmen zur Anwendung kommen, wie z.B. Polyethylen, Ethylvinylacetat etc. Bei der Polyisobutylen-Matrix konnten bei Verwendung von Trägermaterialien mit signifikant unterschiedlichen Wasserdampfdurchlässigkeiten (water vapour transmission rate; WVTR) von 5933,5 g/m^{2*}24h (Viskose) über 1509,9 g/m^{2*}24h (Polyurethan) bis 25,7 g/m^{2*}24h (Polyethylen) keine signifikanten Unterschiede in der Wirkstofffreisetzung festgestellt werden. Ursächlich ist hierbei die Hydrophobie der PIB-Matrix als bestimmender Faktor für die WVTR. Bei Verwendung von hydrophilen Füllstoffen, wie z.B. Cellulose oder Polyacrylsäurederivate, in einer PIB-Matrix können Trägermaterialien unterschiedlicher WVTR als bestimmender Faktor der Wirkstofffreisetzung jedoch wieder relevant werden.

Beim Wassergel kann die Wirkstofffreisetzung durch WVTR des verwendeten Trägermaterials signifikant beeinflusst werden. So zeigten z.B. Muster mit Viskoseträger eine um 14 % höhere Wirkstofffreisetzung gegenüber Mustern mit PU-Trägern.

Da die erfindungsgemäßen Wassergel- wie auch Polyisobutylen-Matrices transparent bis maximal translucent sind, werden mit den vorstehend beschriebenen Folien als Trägermaterialien kaschierten Patches optisch kaum wahrgenommen und können daher auch über längere Zeiträume unauffällig angewendet werden. Durch eine entsprechende Einfärbung der Matrices oder Abdeckung mit einem vorgefärbten Träger kann ein erfindungsgemäßes Patch aber auch in unauffälliger Hauttönung hergestellt werden.

Erfindungsgemäße selbstklebende Patches können jedwede beliebige Form und Größe haben, z.B. rund, rechteckig, quadratisch etc. Besonders vorteilhaft ist, das erfindungsgemäße Patches auf PIB- wie auch auf WG-Basis vom Anwender mittels handelsüblicher Scheren in jedwede beliebige Form und Größe zu Recht geschnitten werden können, um das Patch dem gewünschten Behandlungsareal exakt anzupassen.

Es hat sich gezeigt, dass die erfindungsgemäßen Wassergel- wie auch Polyisobutylen-Matrices insbesondere zur Behandlung von lokalen Hyperpigmentierungen wie Altersflecken, Sommersprossen, postinflammatorischen Hyperpigmentierungen (z.B. in Folge von *Pseudofollikulitis barbae*) als besonders gut handhabbar und wirksam erwiesen haben, wenn diese in runder bis ovaler Form mit einem Durchmesser von ≤ 20mm, bevorzugt ≤ 15mm, ganz besonders bevorzugt ≤ 10mm vorlagen.

Es hat sich gezeigt, dass die erfindungsgemäßen Wassergel- wie auch Polyisobutylen-Matrices insbesondere zur Behandlung von lokalen Hyperpigmentierungen wie Melasma als besonders gut handhabbar und wirksam erwiesen haben, wenn diese eine Fläche ≥ 25cm², bevorzugt ≥ 15cm² ganz besonders bevorzugt ≥ 4 cm² aufwiesen.

### Beispiele:

**Beispiel 1; feucht klebender Film auf Basis Polyacrylsäure / Polyvinylalkohol (FKF)**

| | Gew.-% |
|---|---|
| Polyvinylalkohol | 68,0 |
| Polyacrylsäure | 16,5 |
| Polyethylenglykol 400 | 9,5 |
| Glycerin | 5,0 |
| 4-n-Butylresorcin | 1,0 |

**Beispiel 2; polare wasserfreie Gelmatrix, Basis Polyacrylsäure / Polyvinylpyrrolidon (WFG)**

| | Gew.-% |
|---|---|
| Dexpanthenol | 3,0 |
| Propandiol | 5,0 |
| Polyethylenglykol 400 | 18,0 |
| Polyacrylsäure | 22,5 |
| Polyvinylpyrrolidon | 3,5 |
| Siliziumdioxid | 4,0 |
| Glycerin | 43,0 |
| 4-n-Butylresorcin | 1,0 |

**Beispiel 3; unpolare Polyisobutylenmatrix (PIB)**

| | Gew.-% |
|---|---|
| PIB 12 | 21,5 |
| PIB 80 | 20,0 |
| PIB 12 | 10,0 |
| Cellulose | 33,0 |
| Isopropylmyristat | 5,0 |
| Ölsäuredecylester | 9,5 |
| 4-n-Butylresorcin | 1,0 |

**Beispiel 4; polare Wassergelmatrix auf Basis Agar Agar / Polyacrylsäure (WG).**

| | Gew.-% |
|---|---|
| Wasser | 49,1 |
| Sorbitol | 15,7 |
| Agar Agar | 2,0 |
| Glycerin | 20,0 |
| Polyacrylsäure | 8,0 |
| NaOH 45% | 4,2 |
| 4-n-Butylresorcin | 1,0 |

## Patentansprüche

1. Transdermale, therapeutische, selbstklebende flächige Bandagen, in Form kosmetischer oder medizinischer Pflaster, welche als Wirkstoff 4-n-Butylresorcin enthalten.

2. Transdermale therapeutische Systeme, selbstklebende flächige Bandagen, insbesondere kosmetische und medizinische Pflaster nach Anspruch 1, **dadurch gekennzeichnet, dass** sie als Matrixsysteme vorliegen.

3. Transdermale therapeutische Systeme, selbstklebende flächige Bandagen, insbesondere kosmetische und medizinische Pflaster nach Anspruch 2, **dadurch gekennzeichnet, dass** die Matrix gewählt wird aus
den unpolaren Matrices auf Basis von Synthese- und Naturkautschuk (KA) den polaren feucht klebenden Film auf Basis Polyacrylsäure / Polyvinylalkohol (FKF) den unpolaren Matrices auf Basis von Polyacrylsäurecopolymeren (PAC) den polaren wasserfreien Gelmatrices auf Basis Polyacrylsäure / Polyvinylpyrrolidon (WFG)
den unpolaren Polyisobutylenmatrices (PIB)
den polaren Wassergelmatrix auf Basis Agar Agar / Polyacrylsäure (WG).

4. Transdermale therapeutische Systeme, selbstklebende flächige Bandagen, insbesondere kosmetische und medizinische Pflaster nach einem der vorstehenden Ansprüche, **dadurch gekennzeichnet, dass** sie 0,001 - 10 Gew.-%, besonders bevorzugt 0,01 - 1 Gew.-%, an 4-n-Butylresorcin enthalten, bezogen auf die Gesamtzusammensetzung der Zubereitungen.

5. Transdermale therapeutische Systeme, selbstklebende flächige Bandagen, insbesondere kosmetische und medizinische Pflaster nach einem der vorstehenden Ansprüche, **dadurch gekennzeichnet, dass** die Zubereitungen 0,001 - 10 Gew.-%, besonders bevorzugt 0,01 - 7,5 Gew.-%, ganz besonders bevorzugt 0,1-5 Gew.-% an Penetrationsbeschleuniger, insbesondere Isopropylpalmitat und/oder Isopropylmyristat enthalten, bezogen auf die Gesamtzusammensetzung der Zubereitungen.

6. Transdermale therapeutische Systeme nach einem der vorstehenden Ansprüche, **gekennzeichnet durch** eine Wirkstofffreisetzung von 4-n-Butylresorcin > 30 % in 24 h, bevorzugt 55 - 65 % in 24 h.

7. Transdermale therapeutische Systeme, selbstklebende flächige Bandagen, insbesondere kosmetische und medizinische Pflaster nach einem der vorstehenden Ansprüche zur Behandlung von lokalen Pigmentierungsstörungen, insbesondere lokalen Hyperpigmentierungen wie Altersflecken, Sommersprossen, postinflammatorischen Hyperpigmentierungen (z.B. in Folge von *Pseudofollikulitis barbae*), Melasma.

## Claims

1. Transdermal therapeutic self-adhesive flat bandages, in the form of cosmetic or medical plasters, which contain 4-n-butylresorcinol as active ingredient.

2. Transdermal therapeutic systems, self-adhesive flat bandages, in particular cosmetic and medical plasters according to Claim 1, **characterized in that** they are in the form of matrix systems.

3. Transdermal therapeutic systems, self-adhesive flat bandages, in particular cosmetic and medical plasters according to Claim 2, **characterized in that** the matrix is selected from
the nonpolar matrices based on synthetic and natural rubber (KA)
the polar wet adhesive film based on polyacrylic acid/polyvinyl alcohol (FKF)
the nonpolar matrices based on polyacrylic acid copolymers (PAC)
the polar anhydrous gel matrices based on polyacrylic acid/polyvinylpyrrolidone (WFG)
the nonpolar polyisobutylene matrices (PIB)
the polar water gel matrix based on agar agar/polyacrylic acid (WG).

4. Transdermal therapeutic systems, self-adhesive flat bandages, in particular cosmetic and medical plasters according to any one of the preceding claims, **characterized in that** they comprise 0.001-10% by weight, particularly preferably 0.01-1% by weight, of 4-n-butylresorcinol, based on the total composition of the preparations.

5. Transdermal therapeutic systems, self-adhesive flat bandages, in particular cosmetic and medical plasters according to any one of the preceding claims, **characterized in that** the preparations comprise 0.001-10% by weight, particularly preferably 0.01-7.5% by weight, very particularly preferably 0.1-5% by weight, of penetration accelerator, in particular isopropyl palmitate and/or isopropyl myristate, based on the total composition of the preparations.

6. Transdermal therapeutic systems, according to any one of the preceding claims, **characterized by** an active ingredient release of 4-n-butylresorcinol > 30% in 24 h, preferably 55-65% in 24 h.

7. Transdermal therapeutic systems, self-adhesive flat bandages, in particular cosmetic and medical plasters according to any one of the preceding claims for the treatment of local pigmentation disorders, in particular local hyperpigmentations such as age spots, freckles, post-inflammatory hyperpigmentations (e.g. as a result of *Pseudofollikulitis barbae*), melasma.

## Revendications

1. Bandages plats autocollants thérapeutiques transdermiques, sous la forme de pansements cosmétiques ou médicaux, qui contiennent de la 4-n-butylrésorcine en tant qu'agent actif.

2. Systèmes thérapeutiques transdermiques, bandages plats autocollants, notamment pansements cosmétiques et médicaux selon la revendication 1, **caractérisés en ce qu'**ils se présentent sous la forme de systèmes de matrice.

3. Systèmes thérapeutiques transdermiques, bandages plats autocollants, notamment pansements cosmétiques et médicaux selon la revendication 2, **caractérisés en ce que** la matrice est choisie parmi
les matrices apolaires à base de caoutchouc de synthèse et naturel (KA),
le film polaire adhérent à l'état humide à base d'acide polyacrylique/alcool polyvinylique (FKF),
les matrices apolaires à base de copolymères de l'acide polyacrylique (PAC),
les matrices de gel anhydres polaires à base d'acide polyacrylique/polyvinylpyrrolidone (WFG),
les matrices de polyisobutylène apolaires (PIB),
la matrice de gel d'eau polaire à base d'agar-agar/acide polyacrylique (WG).

4. Systèmes thérapeutiques transdermiques, bandages plats autocollants, notamment pansements cosmétiques et médicaux selon l'une quelconque des revendications précédentes, **caractérisés en ce qu'**ils contiennent 0,001 à 10 % en poids, de manière particulièrement préférée 0,01 à 1 % en poids, de 4-n-butylrésorcine, par rapport à la composition totale des préparations.

5. Systèmes thérapeutiques transdermiques, bandages plats autocollants, notamment pansements cosmétiques et médicaux selon l'une quelconque des revendications précédentes, **caractérisés en ce que** les préparations contiennent 0,001 à 10 % en poids, de manière particulièrement préférée 0,01 à 7,5 % en poids, de manière tout particulièrement préférée 0,1 à 5 % en poids, d'accélérateurs de pénétration, notamment de palmitate d'isopropyle et/ou de myristate d'isopropyle, par rapport à la composition totale des préparations.

6. Systèmes thérapeutiques transdermiques selon l'une quelconque des revendications précédentes, **caractérisés par** une libération d'agent actif de 4-n-butylrésorcine > 30 % en 24 h, de préférence de 55 à 65 % en 24 h.

7. Systèmes thérapeutiques transdermiques, bandages plats autocollants, notamment pansements cosmétiques et médicaux selon l'une quelconque des revendications précédentes, pour le traitement de troubles locaux de la pigmentation, notamment d'hyperpigmentations locales telles que les taches de vieillesse, les taches de rousseur, les hyperpigmentations post-inflammatoires (p. ex. en conséquence de *Pseudofolliculitis barbae*), le mélasma.
